# EUROPEAN PATENT APPLICATION

(11) **EP 3 695 771 A1**
(43) Date of publication of application: **19.08.2020**
(21) Application number: 20155493.8
(22) Date of filing: 04.02.2020
(51) Int. Cl.: A61B 1/00, A61B 1/07

(54) **ILLUMINATION OPTICAL DEVICE AND ENDOSCOPE**

(30) Priority: 12.02.2019 JP 2019022835
(71) Applicant: Fujifilm Corporation, Minato-ku Tokyo 106-8620 (JP)
(72) Inventor: OHASHI, Eiji, Ashigarakami-gun Kanagawa (JP)
(74) Representative: Klunker IP Patentanwälte PartG mbB

(57) **Abstract**

There are provided an illumination optical device and an endoscope that can suppress the temperature rise of a distal end portion of an endoscope.

An illumination optical device is mounted in a distal end portion of an endoscope. The illumination optical device is adapted so that an optical rod, a first lens, a spacer ring, and a second lens are received on the inner periphery of a lens barrel in this order from the rear end side toward the distal end side. The lens barrel is made of metal and polishing is performed on the inner periphery of the lens barrel, so that reflectance is increased. Since reflectance on the inner periphery of the lens barrel is purposely increased in this way on the contrary to the related art, temperature rise can be suppressed. Further, since the illumination optical device has been designed in the related art on the premise that reflected light exists, design does not need to be significantly changed even in a case where reflectance is to be increased.

## Description

### BACKGROUND OF THE INVENTION

### 1. Field of the Invention

The present invention relates to an illumination optical device and an endoscope that irradiate a portion to be observed with illumination light generated from a light source.

### 2. Description of the Related Art

A medical endoscope includes an observation optical device and an illumination optical device that are built in the distal end portion of an insertion part to be inserted into a human body, illuminates a portion to be observed by the illumination optical device, obtains information about the image of the illuminated portion to be observed as video signals by the observation optical device, and displays the image on a monitor or the like. A light guide is received in an interior space that passes through the insertion part from the distal end portion to the proximal end portion. The light guide is, for example, a bundle of a plurality of optical fibers, and is adapted so that illumination light is incident on one end (incident end) of the light guide from an external light source device, is guided to the distal end portion of the light guide, and is emitted from the other end (emission end) of the light guide. The illumination light emitted from the emission end is applied to a portion to be observed through the illumination optical device. The illumination optical device includes one or a plurality of optical elements, such as lenses, and a lens barrel in which the optical elements are received. The illumination optical device makes the illumination light, which is emitted from the light guide, converge and/or be diffused so that the illumination light to be applied to the portion to be observed is distributed in a desired manner.

Since illumination light is made to converge and/or be diffused in the lens barrel of the illumination optical device as described above, the illumination light is applied to and absorbed by the inner periphery of the lens barrel. As a result, there is a problem that a temperature rises. For this reason, JP5345171B discloses an illumination optical device of which the inner diameter is increased toward a distal end side. According to this, the quantity of illumination light applied to and absorbed by the inner periphery of the lens barrel can be reduced, so that temperature rise can be suppressed.

### SUMMARY OF THE INVENTION

According to the device disclosed in JP5345171B, temperature rise can be suppressed as described above but the quantity of illumination light has been increased in recent years for the purpose of obtaining a clearer observation image or the like. Accordingly, temperature rise needs to be more reliably suppressed.

The invention has been made in consideration of the above-mentioned problems, and an object of the invention is to provide an illumination optical device and an endoscope that can more reliably suppress temperature rise.

To achieve the object, there is provided an illumination optical device according to an aspect of the invention that is provided in a distal end portion of an insertion part of an endoscope and irradiates a portion to be observed with illumination light generated from a light source device and transmitted to the distal end portion through a light guide. The illumination optical device comprises an optical element on which the illumination light transmitted from the light guide is incident and which emits the incident illumination light to the portion to be observed and a lens barrel that is formed in a tubular shape and supports a side surface of the optical element by an inner periphery thereof, and at least a portion of the inner periphery of the lens barrel facing the side surface of the optical element is made of polished metal.

Reflectance of the illumination light on at least the portion of the inner periphery of the lens barrel facing the side surface of the optical element may be 85% or more.

The optical element may adhere to the inner periphery with an adhesive having a light-transmitting property.

Reflectance of the illumination light on the inner periphery including the adhesive may be 85% or more.

The inner periphery may be made of aluminum.

The inner periphery may be made of stainless steel.

The optical element may include a first element that is disposed on a front side of the light guide and a first lens that is disposed on a front side of the first element.

The optical element may include a second lens that is disposed on a front side of the first lens.

The optical element may be formed of one element that is disposed on a front side of the light guide, and a light incident surface of the optical element may be a light diffusion surface that diffuses the illumination light transmitted from the light guide.

Further, to achieve the object, there is provided an endoscope according to another aspect of the invention comprising an illumination optical device that irradiates a portion to be observed with illumination light generated from a light source device and transmitted to a distal end portion of an insertion part through a light guide and is provided in the distal end portion. The illumination optical device includes an optical element on which the illumination light transmitted from the light guide is incident and which emits the incident illumination light to the portion to be observed and a lens barrel that is formed in a tubular shape and supports a side surface of the optical element by an inner periphery thereof, and at least a portion of the inner periphery of the lens barrel facing the side surface of the optical element is made of polished metal.

According to the aspect of the invention, a portion of the inner periphery of the lens barrel is made of polished metal, so that reflectance on the inner periphery of the lens barrel is improved. Accordingly, temperature rise can be more reliably suppressed.

### BRIEF DESCRIPTION OF THE DRAWINGS

Fig. 1 is a schematic diagram of an endoscope apparatus.
Fig. 2 is an exploded perspective view of a distal end portion of an endoscope and illumination optical devices.
Fig. 3 is a cross-sectional view of the distal end portion of the endoscope and the illumination optical device.
Fig. 4 is a diagram showing a ratio of the loss of illumination light at each portion.
Fig. 5 is a diagram showing the sums of the losses of light quantity and heat generation ratios of Example and Comparative Examples.
Fig. 6 is a diagram showing the lens efficiency and the sums of the losses of light quantity of Example and Comparative Examples.
Fig. 7 is a diagram showing heat generation ratios of Example and Comparative Examples.
Fig. 8 is a diagram showing a relationship between reflectance and a heat generation ratio.
Fig. 9 is a diagram showing a relationship between surface roughness and reflectance.
Fig. 10 is a cross-sectional view of the distal end portion of the endoscope and an illumination optical device.

### DESCRIPTION OF THE PREFERRED EMBODIMENTS

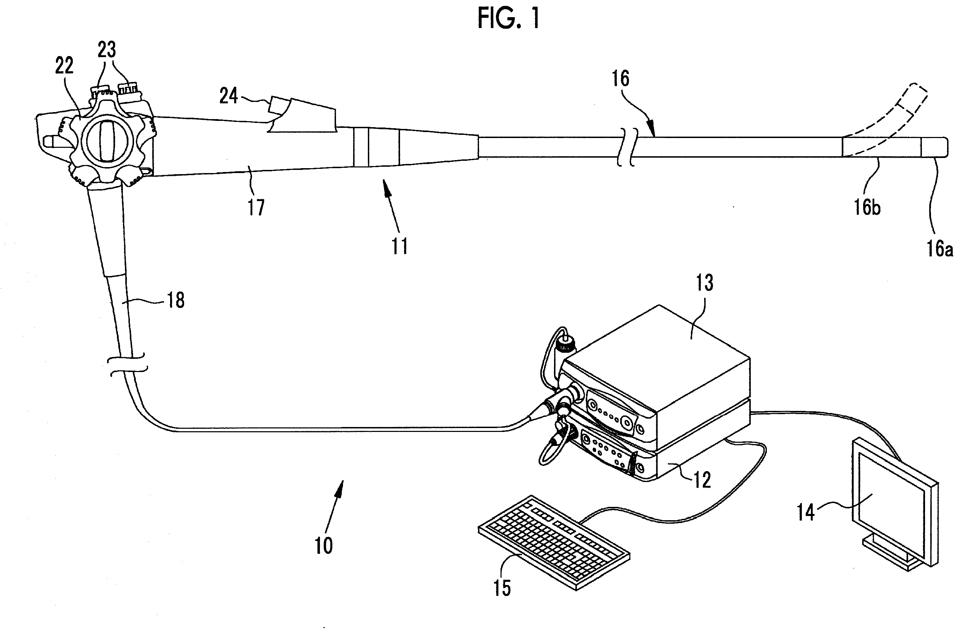

As shown in Fig. 1, an endoscope apparatus 10 comprises an endoscope 11, a processor device 12, a light source device 13, a monitor 14, and a console 15. The endoscope 11 picks up the image of a subject. The processor device 12 performs the control of the endoscope apparatus 10, image processing, and the like. The light source device 13 generates illumination light. The monitor 14 is a display unit that displays an endoscopic image. The console 15 is an input device used to perform setting input for the processor device 12 and the like.

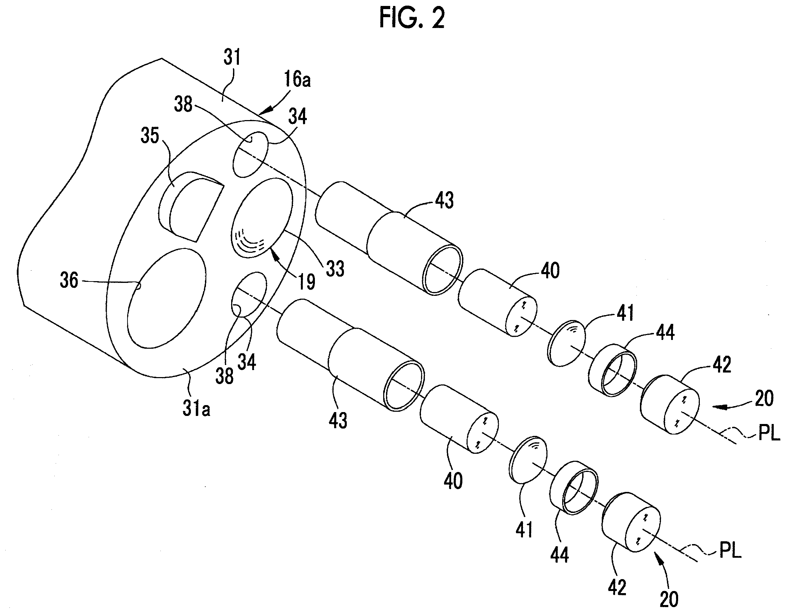

The endoscope 11 comprises a flexible insertion part 16 that is to be inserted into an object to be examined, an operation part 17 that is connected to the proximal end portion of the insertion part 16 and is used for the grip of the endoscope 11 and the operation of the insertion part 16, and a universal cord 18 that connects the operation part 17 to the processor device 12 and the light source device 13. An image pickup element, an objective optical system 19, and a pair of illumination optical devices 20 are built in a distal end portion 16a that is a distal end portion of the insertion part 16 (see Fig. 2). Further, a bendable portion 16b, which can be freely bent, is connected to the rear end of the distal end portion 16a.

The operation part 17 is provided with angle knobs 22, operation buttons 23, and a forceps inlet 24. The angle knobs 22 are rotationally operated to adjust the bending direction and the degree of bend of the insertion part 16. The operation buttons 23 are used for various operations, such as the supply of air, the supply of water, and suction. The forceps inlet 24 communicates with a forceps channel that is inserted into the insertion part 16. An air/water supply channel, a signal cable, and the like are inserted into the universal cord 18.

The processor device 12 is electrically connected to the endoscope 11 and the light source device 13, and generally controls the operation of the endoscope apparatus 10. The processor device 12 controls the drive of the image pickup element of the distal end portion 16a through the signal cable that is inserted into the universal cord 18 and the insertion part 16. Further, the processor device 12 acquires image pickup signals output from the image pickup element through the signal cable, performs various kinds of image processing on the image pickup signals, and displays the image pickup signals on the monitor 14 as an observation image.

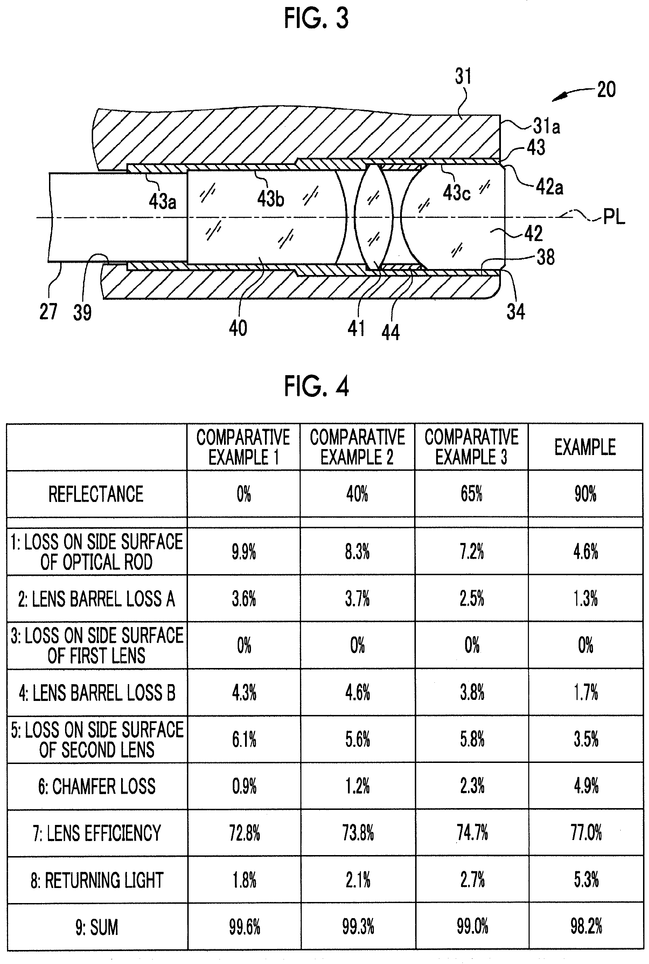

A light source for illumination light is built in the light source device 13. Illumination light generated from the light source device 13 is guided to the illumination optical devices 20 through light guides 27 (see Fig. 3), and a portion to be observed is irradiated with the illumination light emitted from the illumination optical devices 20. Each of the light guides 27 is, for example, a columnar bundle of a plurality of optical fibers (fiber wires), and is inserted into the universal cord 18 and the insertion part 16 so that emission-side end portions of the light guides 27 reach the distal end portion 16a. Illumination light generated from the light source is incident on the end faces of the light guides 27 facing the light source device 13, and the light guides 27 guide the illumination light having been incident to the distal end portion 16a and emit the illumination light from the end face thereof facing the distal end portion 16a.

As shown in Fig. 2, the distal end portion 16a includes a columnar distal-end-portion body 31, the image pickup element, the objective optical system 19, and the pair of illumination optical devices 20 that are assembled in the distal-end-portion body 31, and the like. The distal-end-portion body 31 is made of a hard resin, a metal material, or the like. An observation window 33, a pair of illumination windows 34, an air/water supply nozzle 35, and a forceps outlet 36, which serves as the outlet of the forceps channel, are provided on a distal end face 31a of the distal-end-portion body 31.

The observation window 33 is used to pick up the image of a portion to be observed, the objective optical system 19 is assembled in the observation window 33, and a part of the observation window 33 is exposed to the outside. The image pickup element is disposed on the inner side of the objective optical system 19, and the image of the portion to be observed is picked up through the objective optical system 19 by the image pickup element.

The illumination windows 34 are used to illuminate the portion to be observed, and the illumination optical devices 20 are assembled in the illumination windows 34. The illumination optical devices 20 distribute illumination light emitted from the light guides 27 so that the illumination light is suitable for image pickup and an image pickup range is uniformly illuminated. The pair of illumination windows 34 is provided on both sides of the observation window 33, but the number and the arrangement of the illumination windows 34 can be appropriately changed.

Each of the illumination optical devices 20 is assembled by being inserted into an illumination-optical-device-mounting hole 38 formed in the distal-end-portion body 31. The illumination-optical-device-mounting holes 38 are formed so as to extend rearward from the distal end face 31a along optical axes PL of the illumination optical devices 20, and the openings of the illumination-optical-device-mounting holes 38 on the distal end face 31a are the illumination windows 34. Further, light guide-insertion holes 39 (see Fig. 3) into which the light guides 27 are inserted are formed in the distal-end-portion body 31 on the inner side of the illumination-optical-device-mounting holes 38 so as to be connected to the illumination-optical-device-mounting holes 38. Both of the illumination-optical-device-mounting hole 38 and the light guide-insertion hole 39 are holes having a circular cross section, and are formed coaxially with each other.

The illumination optical devices 20 comprises an optical rod (an optical element, a first element) 40, a first lens (optical element) 41, a second lens (optical element) 42, a lens barrel 43, and a spacer ring 44. Further, each of the illumination optical devices 20 is adapted so that the optical rod 40, the first lens 41, the spacer ring 44, and the second lens 42 are received on the inner periphery of the lens barrel 43 in this order from the rear end side (light guide side) toward the distal end side (illumination window side). The optical rod 40, the first lens 41, the spacer ring 44, and the second lens 42 may adhere to the inner periphery of the lens barrel 43 with an adhesive.

The inner periphery of the lens barrel 43 is increased in diameter by two stages from the rear end side toward the distal end side, so that a first inner diameter portion 43a having the smallest inner diameter is formed on the rear end side, a second inner diameter portion 43b having an inner diameter larger than the inner diameter of the first inner diameter portion 43a is formed at an intermediate portion, and a third inner diameter portion 43c having the largest inner diameter is formed on the distal end side.

The inner diameter of the first inner diameter portion 43a is smaller than the outer diameter of the optical rod 40 (for example, smaller than the outer diameter of the optical rod 40 by about 0.1 mm), and the inner diameter of the second inner diameter portion 43b is slightly larger than the outer diameter of the optical rod 40 (for example, larger than the outer diameter of the optical rod 40 by about 10 µm). The optical rod 40 is inserted into the second inner diameter portion 43b from the distal end side of the lens barrel 43. Then, the rear end of the optical rod 40 touches a step formed between the first inner diameter portion 43a and the second inner diameter portion 43b, so that the optical rod 40 is positioned in a front-rear direction, and the side surface of the optical rod 40 is held by the inner periphery of the second inner diameter portion 43b.

The inner diameter of the second inner diameter portion 43b is smaller than the outer diameter of the first lens 41 (for example, smaller than the outer diameter of the first lens 41 by about 0.1 mm), and the inner diameter of the third inner diameter portion 43c is slightly larger than the outer diameter of the first lens 41 (for example, larger than the outer diameter of the first lens 41 by about 10 µm). Further, the length of the second inner diameter portion 43b (the length of the second inner diameter portion 43b in the direction of the optical axis PL) is longer than the length of the optical rod 40 (the length of the optical rod 40 in the direction of the optical axis PL). The first lens 41 is inserted into the third inner diameter portion 43c from the distal end side of the lens barrel 43 after the optical rod 40 is inserted into the second inner diameter portion 43b. Then, the rear end of the first lens 41 touches a step formed between the second inner diameter portion 43b and the third inner diameter portion 43c, so that the first lens 41 is positioned in a front-rear direction with a predetermined interval between the first lens 41 and the optical rod 40, and the side surface of the first lens 41 is held by the inner periphery of the second inner diameter portion 43b.

The spacer ring 44 and the second lens 42 are formed so that the outer diameter of each of the spacer ring 44 and the second lens 42 is slightly smaller than the inner diameter of the third inner diameter portion 43c (for example, smaller than the inner diameter of the third inner diameter portion 43c by about 10 µm). The spacer ring 44 is inserted into the third inner diameter portion 43c after the first lens 41 is inserted into the third inner diameter portion 43c. After that, the second lens 42 is inserted into the third inner diameter portion 43c. Then, the rear end of the spacer ring 44 touches the first lens 41, so that the spacer ring 44 is positioned in the front-rear direction. Further, the second lens 42 is positioned in the front-rear direction with a predetermined interval between the first lens 41 and the second lens 42 by the spacer ring 44 that is disposed between the first lens 41 and the second lens 42. Furthermore, the side surfaces of the spacer ring 44 and the second lens 42 are held by the inner periphery of the third inner diameter portion 43c.

Further, the inner periphery of the first inner diameter portion 43a is formed to be slightly larger than the outer periphery of the light guide 27 (for example, larger than the outer periphery of the light guide 27 by about 10 µm), and the distal end portion of the light guide 27 is inserted into and supported by the first inner diameter portion 43a in a case where the illumination optical device 20 (lens barrel 43) is mounted in the illumination-optical-device-mounting hole 38. In a case where illumination light emitted from the light guide 27 passes through an optical element (in this embodiment, the optical rod 40, the first lens 41, and the second lens 42), the illumination light is distributed in a desired manner by converging and/or being diffused and is emitted to the portion to be observed. In this embodiment, a chamfer 42a subjected to chamfering (machining for cutting off a corner) is provided on the outer periphery of the distal end portion of the second lens 42 (a portion of the second lens 42 protruding from the distal end face 31a).

Since illumination light passing through the optical element converges and/or is diffused in this way in the illumination optical device 20, illumination light inclined from the optical axis PL and traveling toward the inner periphery of the lens barrel 43 is also generated. In general, due to a concern that this illumination light (illumination light traveling toward the inner periphery of the lens barrel 43) may be an obstacle to obtaining desired light distribution may be obstructed, usually, the color of the inner periphery of the lens barrel 43 tends to be set to black and/or a black adhesive tends to be used as an adhesive used to fix the optical element or the like to the inner periphery of the lens barrel 43, for the purpose of increasing the absorbance (the absorbance of illumination light) of the inner periphery of the lens barrel 43. Further, there is also a case where special consideration is not given for the absorbance of the inner periphery of the lens barrel 43.

Since it is difficult to make absorbance be 100%, it is necessary to consider the reflection of illumination light from the inner periphery of the lens barrel 43 in a step of designing the illumination optical device 20. That is, the illumination optical device 20 is designed on the premise that reflected light exists. Further, there is also a problem that temperature rise is caused by the absorption of illumination light in a case where absorbance is increased. For this reason, the reflectance of illumination light is made to increase in the illumination optical device 20 on the contrary without an increase in the absorbance of illumination light. Specifically, the lens barrel 43 is made of metal (in this embodiment, stainless steel). Further, polishing is performed on the inner periphery of the lens barrel 43. The lens barrel 43 is made of metal and polishing is performed on the inner periphery of the lens barrel 43 as described above to purposely increase reflectance on the inner periphery of the lens barrel 43 (in this embodiment, to make reflectance on the inner periphery of the lens barrel 43 be 85% or more) on the contrary to the related art, so that temperature rise can be suppressed. Furthermore, since the illumination optical device has been designed in the related art on the premise that reflected light exists, design does not need to be significantly changed even in a case where reflectance is to be increased.

Examples of polishing include chemical polishing and electrolytic polishing. Of course, polishing may be performed on the inner periphery of the lens barrel 43 by using an abrasive or the like. Further, the material of the lens barrel may be changed to metal other than stainless steel, for example, aluminum. Furthermore, coating may be performed on the inner periphery of the lens barrel 43 to increase the reflectance of illumination light. Examples of coating include plating. Moreover, the inner periphery of the lens barrel may be coated with aluminum by sputtering or vapor deposition.

Further, the optical element may adhere to the inner periphery of the lens barrel 43 with an adhesive. However, since illumination light is absorbed in this case by the adhesive even though reflectance on the inner periphery of the lens barrel 43 is increased, temperature rise is caused. For this reason, it is preferable that a transparent adhesive (having a light-transmitting property) is used in a case where the optical element is to adhere to the inner periphery of the lens barrel 43 with an adhesive. Furthermore, in a case where the optical element is to adhere to the inner periphery of the lens barrel 43 with an adhesive, it is preferable that reflectance on the inner periphery of the lens barrel 43 including the adhesive is increased, specifically, is set to 85% or more.

Moreover, since a portion, on which reflectance is to be increased, of the inner periphery of the lens barrel 43 can be freely set, reflectance over the entire inner periphery of the lens barrel 43 may be increased or reflectance on a part of the inner periphery of the lens barrel 43 may be increased. However, in a case where reflectance on a part of the inner periphery of the lens barrel 43 is to be increased, it is preferable that reflectance on at least a portion of the inner periphery of the lens barrel 43 facing the side surface of the optical element is increased. In addition, reflectance not only on the inner periphery of the lens barrel 43 but also on the spacer ring 44 (the inner periphery of the spacer ring 44) may be increased.

The results of the verification of the effects of the invention, which is made using a simulation, will be described below with reference to Figs. 4 to 9. As shown in Figs. 4 to 7, the verification is made on the basis of Example 1 of the invention and Comparative Examples 1 to 3 to be compared with Example 1.

Example 1 is an example where the lens barrel 43 is made of aluminum, polishing is performed on the lens barrel 43, and the optical rod 40, the first lens 41, the spacer ring 44, and the second lens 42 adhere to the inner periphery of the lens barrel 43 with an adhesive having a light-transmitting property in the illumination optical device 20 shown in Fig. 3, so that reflectance over the entire inner periphery of the lens barrel 43 including an adhering portion is 90%.

Comparative Example 1 is an example where the lens barrel 43 is made of stainless steel, and the optical rod 40, the first lens 41, the spacer ring 44, and the second lens 42 adhere to the inner periphery of the lens barrel 43 with a black adhesive in the illumination optical device 20 shown in Fig. 3, so that reflectance over the entire inner periphery of the lens barrel 43 including an adhering portion is 0%.

Further, Comparative Example 2 is an example where reflectance over the entire inner periphery of the lens barrel 43 is 40%.

Furthermore, Comparative Example 3 is an example where an adhesive having a light-transmitting property is used as the adhesive in Comparative Example 1, so that reflectance over the entire inner periphery of the lens barrel 43 is 65%.

As shown in Fig. 4, 1 to 9 to be described below are obtained in the verification. Results are as shown in Fig. 4.

### 1: loss on the side surface of the optical rod

The loss on the side surface of the optical rod is the loss of illumination light that is caused by a portion facing the side surface of the optical rod 40 (a ratio of illumination light, which is absorbed by the inner periphery of the lens barrel 43 and is not emitted, to incident illumination light).

### 2: lens barrel loss A

The lens barrel loss A is the loss of illumination light that is caused by a portion between the optical rod 40 and the first lens 41.

### 3: loss on the side surface of the first lens

The loss on the side surface of the first lens is the loss of illumination light that is caused by a portion facing the side surface of the first lens 41.

### 4: lens barrel loss B

The lens barrel loss B is the loss of illumination light that is caused by a portion between the first lens 41 and the second lens 42.

### 5: loss on the side surface of the second lens

The loss on the side surface of the second lens is the loss of illumination light that is caused by a portion facing the side surface of the second lens 42.

### 6: chamfer loss

The chamfer loss is the loss of illumination light that is caused by the chamfer 42a.

### 7: lens efficiency (transmittance)

The lens efficiency (transmittance) is the transmittance of illumination light of the optical element (the optical rod 40, the first lens 41, and the second lens 42).

### 8: light returning to the light guide

The light returning to the light guide is a ratio of illumination light, which returns to the light guide 27, to illumination light that is incident on the illumination optical device 20 from the light guide 27.

### 9: sum

The sum is the value of the sum of 1 to 8 and is substantially equal to the quantity of illumination light incident on the optical element from the light guide 27.

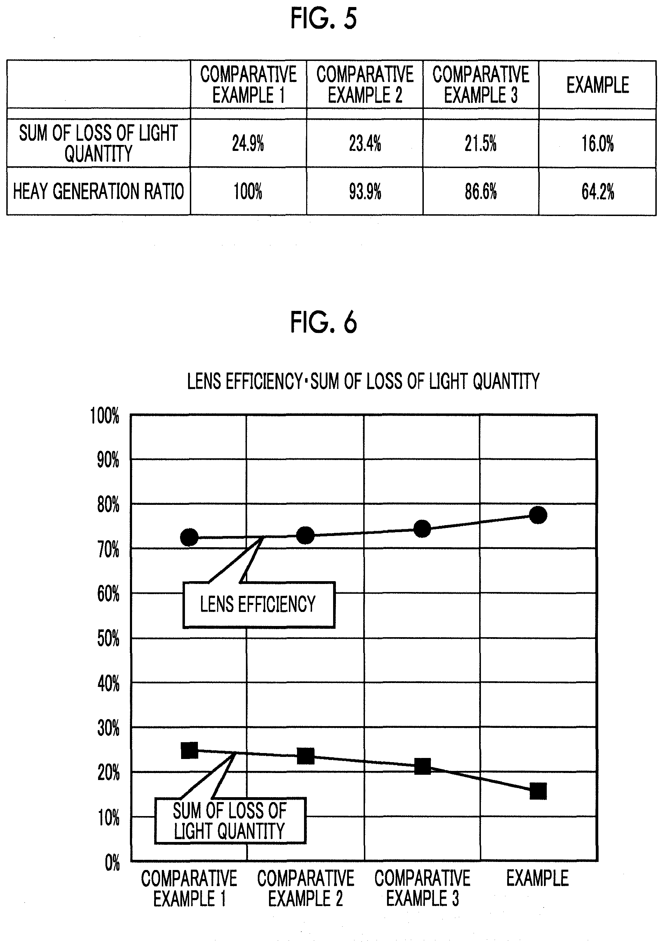

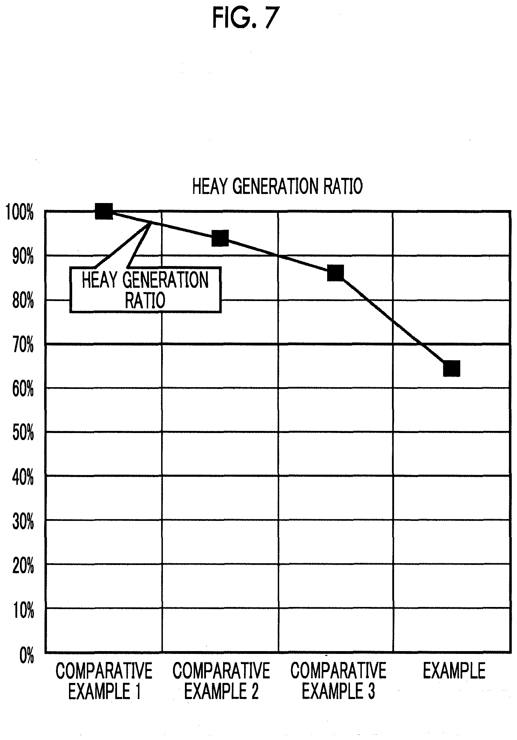

Further, as shown in Figs. 5 to 7, the sums of the losses of light quantity causing temperature rise (the generation of heat) (the sums of 1 to 6) and heat generation ratios (the ratios of the sums of the losses of light quantity of Comparative Examples 2 and 3 and Example in a case where the sum of the losses of light quantity of Comparative Example 1 is assumed as a heat generation ratio of 100%) are obtained in the verification. The sums of the losses of light quantity and heat generation ratios of Example and Comparative Examples 1 to 3 are as shown in Fig. 5. Fig. 6 shows the sums of the losses of light quantity and the lens efficiency (transmittance) (see Fig. 5) of Example and Comparative Examples 1 to 3, and square marks show the sums of the losses of light quantity and circular marks show the lens efficiency (transmittance). Further, Fig. 7 shows heat generation ratios of Example and Comparative Examples 1 to 3. It is found from Figs. 5 and 7 that a target can be achieved in Example since a heat generation ratio is lower than 70% set as the target and targets cannot be achieved in Comparative Examples 1 to 3 other than Example since heat generation ratios are higher than 70%.

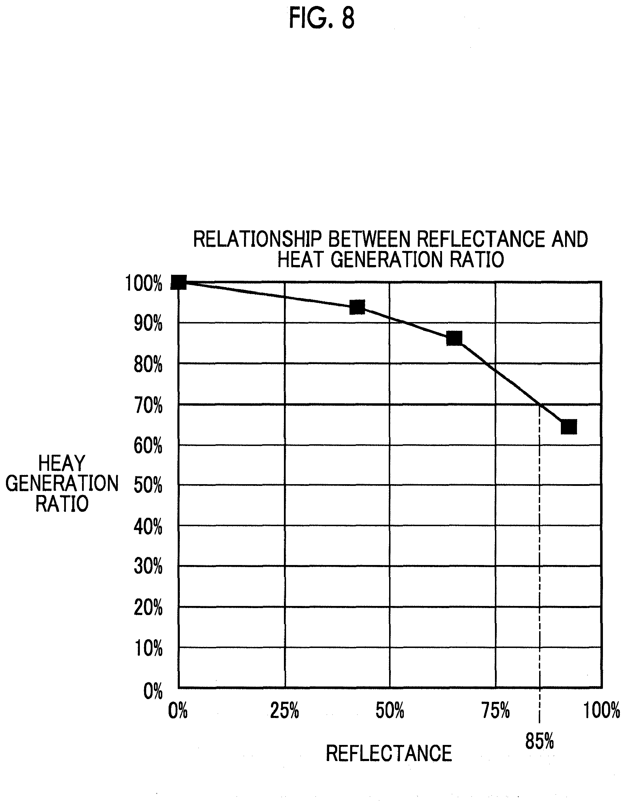

Further, a relationship between reflectance over the entire inner periphery of the lens barrel 43 including an adhering portion and a heat generation ratio is acquired in the verification as shown in Fig. 8. In this verification, the above-mentioned relationship is obtained using the heat generation ratios (see Fig. 7) of Example and Comparative Examples 1 to 3 and reflectance over the entire inner periphery of the lens barrel 43 including an adhering portion of Example and Comparative Examples 1 to 3 (the reflectance is 90% in Example, the reflectance is 0% in Comparative Example 1, the reflectance is 40% in Comparative Example 2, and the reflectance is 65% in Comparative Example 3). It is found from this verification that the target can be achieved since a heat generation ratio is lower than 70% set as the target in a case where reflectance over the entire inner periphery of the lens barrel 43 including an adhering portion is 85% or more.

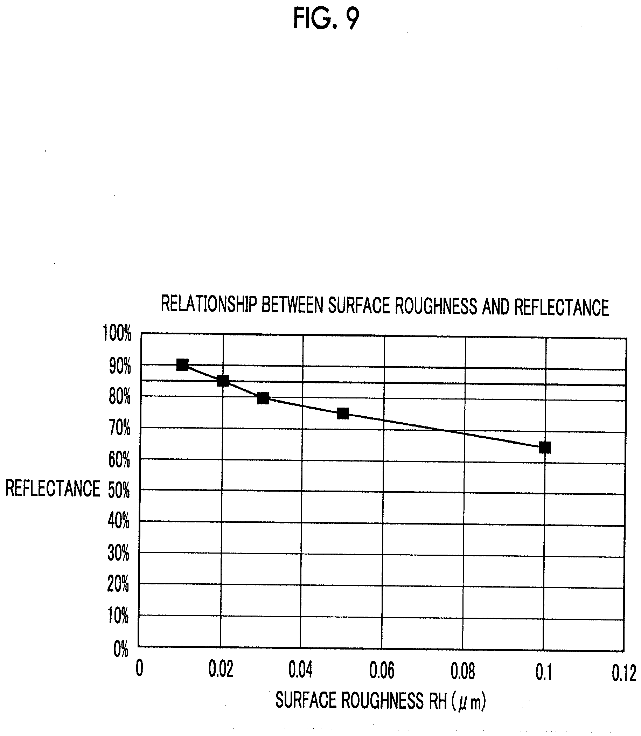

Since it is found from the above-mentioned verification that the target can be achieved in a case where reflectance over the entire inner periphery of the lens barrel 43 including an adhering portion is 85% or more, the surface roughness "Ra" of aluminum at which the target can be achieved in a case where the lens barrel is made of aluminum is also examined subsequently. A relationship between the surface roughness of aluminum and the reflectance is as shown in Fig. 9. Accordingly, it is found that the target can be achieved in a case where "Ra" is 0.02 µm or less.

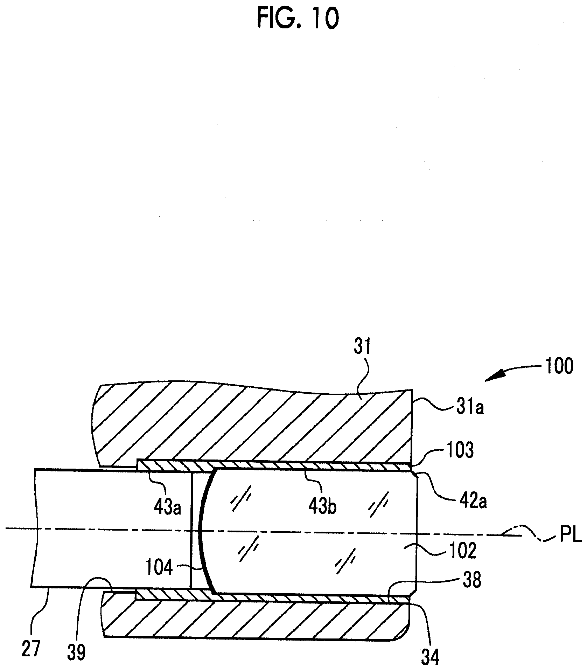

The invention is not limited to the embodiment, and the detailed configuration of the invention can be appropriately changed. For example, an example where the optical element includes the optical rod, the first lens, and the second lens has been described in the embodiment, but the invention is not limited thereto. Since the number, the types, and/or the combination of the elements of the optical element can be appropriately changed without being limited to the embodiment, the optical element may be composed of one element, for example, as in an illumination optical device 100 shown in Fig. 10. In Fig. 10, an optical element of the illumination optical device 100 is only an optical rod 102 and the optical rod 102 is received on the inner periphery of the lens barrel 103. Further, the optical rod 102 includes fine unevenness or the like formed on a light incident surface 104 thereof as compared to the above-mentioned optical rod 40, so that the optical rod 102 is made to have a function to diffuse illumination light on the light incident surface 104. The same members as those of the above-mentioned embodiment are denoted in Fig. 10 by the same reference numerals as those of the above-mentioned embodiment, and the description thereof will be omitted. Of course, the illumination optical device is not limited to the examples shown in Figs. 3 and 10, and the optical element may be composed of two elements or may be composed of four or more elements.

Furthermore, an example where the lens barrel 43 is made of stainless steel or aluminum has been described in the embodiment, but the invention is not limited thereto. The material of the lens barrel 43 can be appropriately changed. In addition, different materials may be used depending on portion so that only the inner peripheral portion of the lens barrel 43 is made of a material different from the material of the other portions of the lens barrel 43. Further, since the distal end portion of the lens barrel 43 is to be inserted into the patient's body, a case where the distal end portion of the lens barrel 43 is made of a highly corrosion-resistant material, such as stainless steel, and the other portions thereof are made of aluminum or the like is also considered. Of course, only a portion, which is to be in contact with the outside, may be sealed (covered) with a highly corrosion-resistant material.

### Explanation of References

10: endoscope apparatus
11: endoscope
12: processor device
13: light source device
14: monitor
15: console
16: insertion part
16a: distal end portion
16b: bendable portion
17: operation part
18: universal cord
19: objective optical system
20, 100: illumination optical device
22: angle knob
23: operation button
24: forceps inlet
27: light guide
31: distal-end-portion body
31a: distal end face
33: observation window
34: illumination window
35: air/water supply nozzle
36: forceps outlet
38: illumination-optical-device-mounting hole
39: light guide-insertion hole
40, 102: optical rod (optical element, first element)
41: first lens (optical element)
42: second lens
42a: chamfer
43, 103: lens barrel
43a: first inner diameter portion
43b: second inner diameter portion
43c: third inner diameter portion
44: spacer ring
104: light incident surface

## Claims

1. An illumination optical device that is provided in a distal end portion of an insertion part of an endoscope and irradiates a portion to be observed with illumination light generated from a light source device and transmitted to the distal end portion through a light guide, the illumination optical device comprising:
an optical element on which the illumination light transmitted from the light guide is incident and which emits the incident illumination light to the portion to be observed; and
a lens barrel that is formed in a tubular shape and supports a side surface of the optical element by an inner periphery thereof,
wherein at least a portion of the inner periphery of the lens barrel facing the side surface of the optical element is made of polished metal.

2. The illumination optical device according to claim 1,
wherein reflectance of the illumination light on at least the portion of the inner periphery of the lens barrel facing the side surface of the optical element is 85% or more.

3. The illumination optical device according to claim 1 or 2,
wherein the optical element adheres to the inner periphery with an adhesive having a light-transmitting property.

4. The illumination optical device according to claim 3,
wherein reflectance of the illumination light on the inner periphery including the adhesive is 85% or more.

5. The illumination optical device according to any one of claims 1 to 4,
wherein the inner periphery is made of aluminum.

6. The illumination optical device according to any one of claims 1 to 4,
wherein the inner periphery is made of stainless steel.

7. The illumination optical device according to any one of claims 1 to 6,
wherein the optical element includes a first element that is disposed on a front side of the light guide and a first lens that is disposed on a front side of the first element.

8. The illumination optical device according to claim 7,
wherein the optical element includes a second lens that is disposed on a front side of the first lens.

9. The illumination optical device according to any one of claims 1 to 6,
wherein the optical element is formed of one element that is disposed on a front side of the light guide, and
a light incident surface of the optical element is a light diffusion surface that diffuses the illumination light transmitted from the light guide.

10. An endoscope comprising:
an illumination optical device that irradiates a portion to be observed with illumination light generated from a light source device and transmitted to a distal end portion of an insertion part through a light guide and is provided in the distal end portion,
wherein the illumination optical device includes
an optical element on which the illumination light transmitted from the light guide is incident and which emits the incident illumination light to the portion to be observed, and
a lens barrel that is formed in a tubular shape and supports a side surface of the optical element by an inner periphery thereof, and
at least a portion of the inner periphery of the lens barrel facing the side surface of the optical element is made of polished metal.
